(19) Europäisches Patentamt / European Patent Office / Office européen des brevets

(11) **EP 4 672 247 A1**

(12) **EUROPEAN PATENT APPLICATION**

(43) Date of publication:
**31.12.2025 Bulletin 2026/01**

(21) Application number: **25185381.8**

(22) Date of filing: **26.06.2025**

(51) International Patent Classification (IPC):
**G16B 30/10** (2019.01) **G16B 40/10** (2019.01)
**C12Q 1/6869** (2018.01)

(52) Cooperative Patent Classification (CPC):
**G16B 30/10; G16B 40/10;** C12Q 1/6869

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **26.06.2024 CN 202410841304**

(71) Applicant: **GeneMind Biosciences Company Limited**
**Shenzhen City, 518023 Guangdong (CN)**

(72) Inventor: **LI, Linsen**
**518023 Shenzhen City (CN)**

(74) Representative: **Schiweck Weinzierl Koch Patentanwälte Partnerschaft mbB**
**Ganghoferstraße 68 B**
**80339 München (DE)**

(54) **METHOD AND APPARATUS FOR DETERMINING BASE SEQUENCE OF NUCLEIC ACID TEMPLATE, DEVICE, AND MEDIUM**

(57) The present application discloses a method and apparatus for determining a base sequence of a nucleic acid template, a device, and a medium, and generally relates to the field of data processing. The method includes: processing an image including a feature corresponding to the nucleic acid template, including: determining a signal intensity at each basic unit position in the image, where the image includes a plurality of basic units, the size of the feature corresponding to the nucleic acid template in the image is represented as one or more basic units, and the size of one basic unit is less than or equal to the size of one pixel of the image; detecting, based on the signal intensity at each basic unit position, the type of one or more bases incorporated into the nucleic acid template corresponding to the basic unit position to determine a detected base sequence at each basic unit position; and clustering, based on a similarity between the detected base sequence at each basic unit position and detected base sequences at surrounding basic unit positions thereof, the detected base sequences or the basic unit positions to determine a portion of the base sequence of the nucleic acid template. The present application can improve the sequencing accuracy and sequencing throughput.

```
┌─────────────────────────────────────────────────┐
│ Process an image including a feature corresponding │  S20
│ to the nucleic acid template, including: determine │
│ a signal intensity at each basic unit position in  │
│ the image                                          │
└─────────────────────────────────────────────────┘
                        │
                        ▼
┌─────────────────────────────────────────────────┐
│ Detect, based on the signal intensity at each      │  S40
│ basic unit position, the type of one or more bases │
│ incorporated into the nucleic acid template        │
│ corresponding to the basic unit position to        │
│ determine a detected base sequence at each basic   │
│ unit position                                      │
└─────────────────────────────────────────────────┘
                        │
                        ▼
┌─────────────────────────────────────────────────┐
│ Cluster, based on a similarity between the detected │  S60
│ base sequence at each basic unit position and       │
│ detected base sequences at surrounding basic unit   │
│ positions thereof, the detected base sequences or   │
│ the basic unit positions to determine a portion of  │
│ the base sequence of the nucleic acid template      │
└─────────────────────────────────────────────────┘
```

**FIG. 1**

EP 4 672 247 A1

**Description**

**TECHNICAL FIELD**

The present disclosure generally relates to the field of data processing, and in particular, to a method and apparatus for determining a base sequence of a nucleic acid template, a device, and a medium.

**BACKGROUND**

Sequencing is a method for genetic testing. It generally refers to the determination of the sequence of biopolymers, including nucleic acids such as deoxyribonucleic acid (DNA) and ribonucleic acid (RNA), for example, the determination of the order of nucleotides or bases (adenine (A), guanine (G), thymine (T), uracil (U), and cytosine (C)) of a given nucleic acid fragment.

In sequencing-by-synthesis (SBS)-based platforms, such as Miseq, Nextseq, and Novaseq platforms (ILLUMINA), reversible terminators are used to perform single base extension for sequencing. The automated sequencing processes on sequencing platforms generally include: (i) introducing a sample solution into a flow cell and hybridizing or connecting a nucleic acid molecule of interest (or referred to as a nucleic acid template) with a designated surface or designated surface position of the flow cell (sometimes referred to as a chip), where the designated surface is provided with a probe; (ii) amplifying the nucleic acid molecule of interest to form a plurality of identical polynucleotide molecule clusters; (iii) contacting a DNA polymerase, a sequencing primer, and a reaction substrate (e.g., a nucleotide analog carrying an optically detectable label and a blocking group, which is also referred to as a reversible terminator and sometimes is also referred to as a nucleotide or a base herein) with the nucleic acid molecule of interest in a condition suitable for the polymerization reaction to perform a controllable polymerase chain reaction (single base extension); (iv) optionally removing an unreacted nucleotide analog and detecting an optical signal from the optically detectable label on the surface; (v) cleaving the blocking group and the optically detectable label of the nucleotide analog incorporated into the nucleic acid molecule of interest; and repeating (iii) to (v) multiple times to determine the base type of a corresponding cycle or repetition incorporated into the molecule clusters of interest based on the optical signal detected from the cycle or repetition, thereby achieving the determination of the base order of at least a portion of the nucleic acid molecule of interest.

For another example, sequencing platforms of MGI, such as DNBseq400, perform rolling circle amplification on the nucleic acid molecule of interest in a liquid phase and attach the amplification product to the chip surface to form a DNB nanoball, and then perform steps similar to steps (iii) to (iv) described above multiple times to achieve the determination of at least a portion of the sequence of the nucleic acid molecule of interest.

On some other sequencing platforms, such as single-molecule sequencing platforms, the process of amplifying the reaction signal of the nucleic acid molecule of interest by increasing the copy number of the nucleic acid molecule of interest is generally not involved. For example, the process of automated sequencing on such platforms generally does not undergo the above step (ii) of amplifying the nucleic acid molecule of interest on the surface.

Specifically, step (iv) includes: detecting a reaction signal from a surface by using a microscopic imaging system to acquire an image, where the reaction signal corresponding to a specific chemical feature may be presented as a spot or a point in the image; and performing base calling of an incorporated base based on the detection of the spot or the point in the image, thereby determining the base type of the nucleic acid molecule of interest.

In the prior art, the process of performing base calling based on a signal/image acquired in a sequencing process generally requires the construction of a template (a coordinate system including position information of each nucleic acid template, or a coordinate system including each chemical feature). For example (see CN112288783B), spots or points in the images of multiple cycles are combined to acquire a set of spots or points corresponding to the chemical features, so as to construct the template; and then the template is used to determine the position of a corresponding chemical feature in the image of a specific cycle, so as to achieve the base calling of the cycle. Such base calling methods are based on the construction of specific templates, which generally includes the identification or detection of image features (e.g., spots) corresponding to chemical features on the surface. Thus, the methods may, as limited by the incompleteness of the identification of image features or the incompleteness of the templates constructed based on the identification of such image features, result in a loss of throughput in the base calling, for example, when the templates constructed or used do not truly or completely reflect the chemical features on the surface. Therefore, how to perform base calling or acquire the base sequence of a nucleic acid molecule of interest is a matter of concern.

**SUMMARY**

The present disclosure is intended to solve, to some extent, at least one of the existing technical problems in the prior art or provide at least a useful alternative.

To this end, according to the embodiments of the present disclosure, in a first aspect, provided is a method for determining a base sequence of a nucleic acid template. The method includes: processing an image including a feature corresponding to the nucleic acid template, including: determining a signal intensity at each basic unit in the image, where the image includes a plurality of basic units, the size of the feature corresponding to the nucleic acid template in the image is represented as one or not less than one basic units, and the size of one basic unit is less than or equal to the size of one pixel of the image; detecting, based on the signal intensity at each basic unit position, the type of one or more bases incorporated into the nucleic acid template of the basic unit position to determine a detected base sequence at each basic unit position; and clustering, based on a similarity between the detected base sequence at each basic unit position and detected base sequences at surrounding basic unit positions thereof, the detected base sequences or the basic unit positions to determine a portion of the base sequence of the nucleic acid template.

In a second aspect, the present disclosure provides an apparatus for determining a base sequence of a nucleic acid template. The apparatus includes:

a processing module, configured for processing an image including a feature corresponding to the nucleic acid template, including: determining a signal intensity at each basic unit in the image, where the image includes a plurality of basic units, the size of the feature corresponding to the nucleic acid template in the image is represented as one or more basic units, and the size of one basic unit is less than or equal to the size of one pixel of the image;

a detection module, configured for detecting, based on the signal intensity at each basic unit position, the type of one or more bases incorporated into the nucleic acid template corresponding to the basic unit position to determine a detected base sequence at each basic unit position, where

the detection module is further configured for clustering, based on a similarity between the detected base sequence at each basic unit position and detected base sequences at surrounding basic unit positions thereof, the detected base sequences or the basic unit positions; and

a determination module, configured for determining, based on the clustering result, a portion of the base sequence of the nucleic acid template.

In a third aspect, the embodiments of the present disclosure provide a computer device, including a memory, a processor, and a computer program stored on the memory and operable on the processor, where the processor, when executing the program, executes the method as described in the embodiments of the present disclosure.

In a fourth aspect, the embodiments of the present disclosure provide a computer-readable storage medium having a computer program stored thereon, where the program, when executed by a processor, executes the method as described in the embodiments of the present disclosure.

In a fifth aspect, the embodiments of the present disclosure provide a computer program product, including an instruction, where the instruction, when operated, enables the execution of the method as described in the embodiments of the present disclosure.

The method and the apparatus for determining a base sequence of a nucleic acid template, the device, the storage medium, and/or the product provided in any one of the above aspects and embodiments of the present disclosure, by performing base calling on the nucleic acid template corresponding to each basic unit position of an image, determine the detected base sequence at each basic unit position, and based on the similarity between the detected base sequence at each basic unit position and the detected base sequences at the surrounding basic unit positions thereof, cluster the detected base sequences and the basic unit positions to determine a portion of the base sequence of the nucleic acid template, so as to avoid or reduce the loss or omission of target signal detection caused by weak reaction signals (relatively weak target signals) of certain nucleic acid templates and/or relatively low sensitivity of the used signal detection or acquisition modes, thereby improving the sequencing accuracy and sequencing throughput.

Additional aspects and advantages of the present disclosure will be partially provided in the following description, will partially become apparent from the following description, or will be learned through the practice of the present disclosure.

## BRIEF DESCRIPTION OF THE DRAWINGS

The features, objectives, and advantages of the present disclosure will become apparent and easily understood by reading the following detailed description of the non-limiting embodiments with reference to the drawings.

FIG. 1 is a schematic flowchart of a method for determining a base sequence of a nucleic acid template according to the embodiments of the present disclosure;

FIG. 2 is a schematic flowchart of another method for determining a base sequence of a nucleic acid template according to the embodiments of the present disclosure;

FIG. 3 is a schematic diagram of a matrix of a basic unit according to the embodiments of the present disclosure;

FIG. 4 is a cluster diagram or score map according to the embodiments of the present disclosure;

FIG. 5 is a schematic flowchart of another method for determining a base sequence of a nucleic acid template according to the embodiments of the present disclosure;

FIG. 6 is a structural schematic diagram of an apparatus for determining a base sequence of a nucleic acid template according to the embodiments of the present disclosure; and

FIG. 7 is a structural schematic diagram of a computer device according to the embodiments of the present disclosure.

## DETAILED DESCRIPTION

The present disclosure will be described in further detail below with reference to the drawings and embodiments. It will be appreciated that the specific embodiments described herein are merely intended to illustrate, rather than limit, the present disclosure.

It should be noted that the embodiments and features in the embodiments of the present disclosure may be combined with each other unless conflicting. The present disclosure will be described in detail below with reference to the drawings and in conjunction with the embodiments. Furthermore, in the present disclosure, the term "and/or" is merely an associative relationship for describing associated objects, and represents three possible relationships. For example, A and/or B may denote that: A is present alone, A and B are present simultaneously, and B is present alone. In the present disclosure, the terms "first", "second", and the like are used for descriptive purposes only rather than being construed as indicating or implying relative importance or implicitly indicating the number of indicated technical features. Therefore, features defined with "first" and "second" may explicitly or implicitly include one or more of the features. In the description of the present disclosure, unless otherwise clearly and specifically defined, the term "plurality" means two or more.

As used herein, the term "sequencing" refers to sequence determination, and is used interchangeably with "nucleotide sequencing" to refer to the determination of base order in nucleotide sequences. The sequencing, for example, may include sequencing by synthesis (SBS), DNA sequencing, and/or RNA sequencing; may include long fragment sequencing and/or short fragment sequencing (the long fragment and short fragment are defined relatively; for example, nucleotide molecules longer than 1 Kb, 2 Kb, 5 Kb, or 10 Kb may be referred to as long fragments, and nucleotide molecules shorter than 1 Kb or 800 bp may be referred to as short fragments); and may also include double-end sequencing, single-end sequencing, paired-end sequencing, and/or the like (the double-end sequencing or paired-end sequencing may refer to the reading of any two segments or portions of the same nucleotide molecule that are not completely overlapping).

In the present disclosure, sequencing may be performed through a sequencing platform. For example, the sequencing platform may be selected from, but is not limited to, the Hiseq/Miseq/Nextseq/Novaseq sequencing platform (Illumina), the Ion Torrent platform (Thermo Fisher/Life Technologies), the BGISEQ and MGISEQ/DNBSEQ platforms (BGI), and single-molecule sequencing platforms.

Generally, a sequencing platform can determine the nucleotide/base type at any designated position on a nucleic acid template through one cycle of sequencing, and can determine the sequence of multiple nucleotides/bases on the nucleic acid template through multiple cycles of sequencing. For example, for a sequencing platform based on SBS sequencing, the reaction system includes a sequencing primer, a reaction substrate (a nucleotide or an analog thereof), a polymerase, and a nucleic acid template. The nucleic acid template is bound to the sequencing primer, and each cycle of sequencing includes a process of controllably performing a polymerase chain reaction, or base extension reaction, by contacting the polymerase, the reaction substrate, and the nucleic acid template in a condition suitable for polymerization reaction, linking or binding or incorporating the nucleotide to the nucleic acid template, and acquiring a corresponding reaction signal. After the completion of each cycle of sequencing, the type of the nucleotide linked or bound to or incorporated into the nucleic acid template is determined based on the reaction signal.

In some embodiments, each cycle of sequencing may include one or more base extension reactions. For example, a mixture of all four types of nucleotides (e.g., A, T, C, and G) may be simultaneously added into the reaction system for the base extension reaction and the acquisition of corresponding reaction signals, and one cycle of sequencing includes one base extension reaction; nucleotide pairs (such as A/T, C/G) may be sequentially added into the reaction system for separate base extension reactions and acquisitions of corresponding reaction signals, and one cycle of sequencing includes two base extension reactions; alternatively, four types of nucleotides (e.g., A, T, C, G) may be sequentially added into the reaction system for separate base extension reactions and acquisition of corresponding reaction signals, and one cycle of sequencing includes four base extension reactions. It should be noted that, as will be appreciated by those skilled in the art, after each base extension reaction, a microscopic imaging system can be used to acquire the corresponding reaction signal and form an image to determine the base type at any designated position on the nucleic acid template based on the image.

In some embodiments, the nucleic acid template undergoes amplification (such as bridge amplification or rolling circle amplification) after binding to a sequencing primer to form a plurality of identical polynucleotide molecules or a nucleic acid molecule cluster. In the process of base extension reaction, a microscopic imaging system is used to acquire reaction signals generated on a nucleic acid molecule cluster and form an image, and the intensity of the reaction signals can be characterized by the brightness value or grayscale value (sometimes also referred to as "pixel value" or "pixel grayscale value") at corresponding positions of the reaction signals imaged in the image. In some platforms, such as single-molecule sequencing platforms, the nucleic acid template does not require an amplification process after binding to the sequencing primer. In the process of base extension reaction, a microscopic imaging system is used to acquire reaction signals generated on a nucleic acid template and form an image, and the intensity of the reaction signals can be characterized by the brightness value or grayscale value at corresponding positions of the reaction signals imaged in the image.

However, the influence of the resolution of the microscopic imaging system, the weak reaction signals of the nucleic acid templates (the target signals are relatively weak), and/or the relatively low sensitivity of the used signal detection or acquisition modes can easily lead to the loss or omission of target signal detection, resulting in a far lower sequencing throughput than expected.

The embodiments of the present disclosure provide a method for determining a base sequence of a nucleic acid template. The method, by performing base calling on the nucleic acid template corresponding to each basic unit position of an image, determines the detected base sequence at each basic unit position, and based on the similarity between the detected base sequence at each basic unit position and the detected base sequences at the surrounding basic unit positions thereof, clusters the detected base sequences and the basic unit positions to determine a portion of the base sequence of the nucleic acid template, so as to avoid or reduce the loss or omission of target signal detection caused by weak reaction signals (relatively weak target signals) of certain nucleic acid templates and/or relatively low sensitivity of the used signal detection or acquisition modes, thereby improving the sequencing accuracy and sequencing throughput.

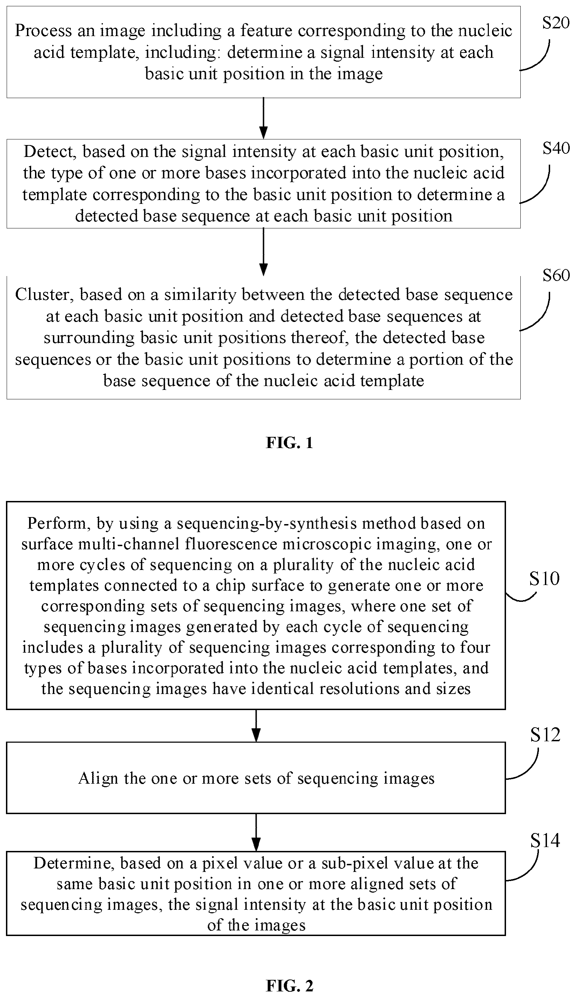

Referring to FIG. 1, the method provided by the embodiments of the present disclosure includes the following steps:

S20, processing an image including a feature corresponding to the nucleic acid template, including: determining a signal intensity at each basic unit position in the image.

The nucleic acid template refers to a nucleic acid molecule of interest.

In some embodiments, the nucleic acid template may be a polymer of nucleotides with a certain length, such as a single-stranded and/or double-stranded nucleic acid molecule, or a complex including single-stranded and/or double-stranded nucleic acid sequences. The nucleotide may be, for example, a ribonucleotide, a deoxyribonucleotide, or an analog or a derivative of a ribonucleotide or a deoxyribonucleotide.

In some examples, the nucleic acid template may be a single-molecule polynucleotide, or a polynucleotide molecule or nucleic acid molecule cluster including a plurality of identical sequences. The single-molecule polynucleotide is, for example, a single-stranded DNA molecule or an RNA molecule. The single-molecule polynucleotide after amplification can form, for example, hundreds or thousands of copies, and the copies can form the polynucleotide molecule or nucleic acid molecule cluster including the plurality of identical sequences described above.

In the embodiments of the present disclosure, the image including a feature corresponding to the nucleic acid template is acquired by photographing using a microscopic imaging system after each base extension reaction during the sequencing of the nucleic acid template. The image is capable of characterizing the feature of the nucleic acid template. The feature of the nucleic acid template may be interpreted as a reaction signal generated when a nucleotide (a reaction substrate) is linked or bound to or incorporated into the nucleic acid template, or may be interpreted as a representation of the reaction signal in the image, or may be interpreted as a fluorescence signal generated after a fluorophore on the nucleic acid template is excited, i.e., a fluorescence signal generated by a fluorophore carried by a nucleotide incorporated into the nucleic acid template through a base extension reaction.

In some embodiments, the image includes a plurality of basic units, and the size of the feature corresponding to one nucleic acid template in the image is represented as one or not less than one basic unit. The size of one basic unit is less than or equal to the size of one pixel of the image. Illustratively, the size of one basic unit is 1 pixel, 0.8 pixels, 0.5 pixels, 0.25 pixels, 0.2 pixels, or 0.1 pixels. The signal intensity at each basic unit position in the image may be characterized by the brightness value or grayscale value at each basic unit position. In a digital image, each pixel has a specific position, which can be represented by coordinates (e.g., Cartesian coordinates). The signal intensity may be represented digitally, for example, using a grayscale value. In a grayscale image, the signal intensity of each pixel is represented as an integer from 0 to 255, which represents different grayscale levels, with lower values indicating darker levels and higher values indicating brighter levels.

In some embodiments, the image including the feature corresponding to the nucleic acid template may be a grayscale image, for example, a 16-bit tiff-format grayscale image of $512 \times 512$ or $2048 \times 2048$. The pixel value of a grayscale image is also generally referred to as the grayscale value. The image may also be a colored image, and the pixel value of each pixel in the colored image is composed of three grayscale pixel values. In the present disclosure, the colored image can be converted into a grayscale image for subsequent processing and detection, so as to reduce the calculation and complexity in image processing. Illustratively, a non-grayscale image may be converted into a grayscale image with methods including, but not limited to, floating point algorithm, integer method, shift method, mean value method, etc.

In the embodiments of the present disclosure, the images including the feature corresponding to the nucleic acid template may be one or more corresponding sets of sequencing images generated in one or more cycles of sequencing, and the sequencing images may be aligned during the processing of step S20, so as to determine the signal intensity at each basic unit position in the image. Specifically, referring to FIG. 2, the method includes the following steps:

S10, performing, by using a sequencing-by-synthesis method based on surface multi-channel fluorescence microscopic imaging, one or more cycles of sequencing on a plurality of the nucleic acid templates connected to a chip surface to generate one or more corresponding sets of sequencing images, where one set of sequencing images generated by each cycle of sequencing includes a plurality of sequencing images corresponding to four types of bases incorporated into the nucleic acid templates, and the sequencing images have identical resolutions and sizes.

In one cycle of sequencing, four types of nucleotides or bases (e.g., A, T, C, G) may be added to the reaction system, and one set of sequencing images can be acquired using a microscopic imaging system to acquire reaction signals generated by each base extension reaction. Illustratively, one set of sequencing images may include a plurality of sequencing images, e.g., four sequencing images, each having the same resolution and including the same number of pixels. In practical applications, a plurality of cycles of sequencing may be performed on the nucleic acid template to acquire a plurality of sets of sequencing images.

S 12, aligning the one or more sets of sequencing images.

In the embodiments of the present disclosure, the alignment of one or more sets of sequencing images, also referred to as the image registration of one or more sets of sequencing images, is a process of precisely aligning the positions of the same scene or object in two or more images. After the alignment of one or more sets of sequencing images, a pixel value or a sub-pixel value can be acquired at the same basic unit position in each sequencing image, such that the signal intensity at the basic unit position is determined based on the pixel values or the sub-pixel values at the basic unit position.

In one embodiment, four types of nucleotides are each provided with a different label (e.g., a fluorophore), and when sequencing is performed, the four types of nucleotides are added into the reaction system as reaction substrates, and the nucleic acid template in the reaction system is subjected to base pairing with the labeled nucleotides under the action of a polymerase. The different labels are excited to emit reaction signals of different colors, and the reaction signal of each color corresponds to one type of nucleotide/base. A set of sequencing images generated by one cycle of sequencing may be a plurality of sequencing images generated from the separate acquisition of each reaction signal by a microscopic imaging system. Illustratively, the set of sequencing images includes a first sequencing image, a second sequencing image, a third sequencing image, and a fourth sequencing image. The first sequencing image, the second sequencing image, the third sequencing image, and the fourth sequencing image are generated by the acquisition of corresponding reaction signals. Aligning the set of sequencing images may refer to processing the set of images such that the features of corresponding nucleic acid templates included in the set of sequencing images are positioned in the same coordinate system.

In some embodiments, aligning the set of sequencing images includes: converting, by taking any one of the sequencing images in the set of sequencing images as a baseline, the coordinate systems of other sequencing images in the set of sequencing images, such that the coordinate systems of the one or more sets of sequencing images are identical, thereby achieving the alignment of the one or more sets of sequencing images. For example, by taking the coordinate system of the first sequencing image as the baseline, the coordinate systems of the second sequencing image, the third sequencing image, and the fourth sequencing image are converted, such that the coordinate systems of the set of sequencing images are identical.

The method for converting the coordinate system used for aligning the sequencing images is not limited in the embodiments of the present disclosure. For example, the alignment of each sequencing image may be performed using the correlation function in MatLab.

In one implementation mode, the four types of nucleotides carry a first label, a second label, a third label, and a fourth label, respectively. For example, four fluorophores with different emission spectra or incompletely overlapped emission spectra; in one cycle of sequencing: a first laser is used for exciting nucleotides, and two of the four types of nucleotides emit a first signal and a second signal, respectively; the microscopic imaging system includes a first camera and a second camera, and the first camera and the second camera synchronously operate to acquire the first signal and the second signal, respectively, so as to acquire the first sequencing image and the second sequencing image; a second laser is used for exciting nucleotides, the other two nucleotides of the four nucleotides emit a third signal and a fourth signal, respectively, and the first camera and the second camera synchronously operate to acquire the third signal and the fourth signal, respectively, so as to acquire the third sequencing image and the fourth sequencing image. The first laser and the second laser may be from two laser devices capable of emitting different wavelengths, or may be from one laser device capable of emitting multiple wavelengths.

Specifically, in one cycle of sequencing, four sequencing images of one field of view come from four wavebands of two cameras. Although optical adjustment is performed as much as possible, pixel offset (chromatic aberration) may still exist between the four sequencing images. Generally, if the optical setting is unchanged, offset caused by corresponding chromatic aberration can be considered to be fixed. If the set of images comes from the first cycle of sequencing (cycle 1) or the first several cycles of sequencing, generally there's no crosstalk or insignificant crosstalk between two designated ones of the four signals corresponding to the four types of bases in the cycle 1 or the first several cycles of sequencing. For example, A, T, G, and C carry fluorescent dyes ATTO-532, ROX, CY5, and IF700, respectively, and in any of the first several cycles of sequencing, at one time point, the first camera photographs A and the second camera photographs G at the same time, and at another time point, the first camera photographs T and the second camera photographs C at the same time; according to the sequencing images/signals acquired in that cycle, generally there's crosstalk between the A and T signals or between the G and C signals, but there's no crosstalk or insignificant crosstalk between the C and T signals or between the A and G signals; with regard to no crosstalk or insignificant crosstalk between the C and T signals, it is shown as that the T signal will not be acquired at a certain position when the C signal is acquired there (T is not bright when C is bright), and thus, in a certain sequencing, it is generally difficult to determine the fixed offset with sequencing images from one cycle of the first several cycles of sequencing.

Therefore, in some examples, aligning a set of sequencing images includes using sequencing images from cycle M of sequencing to perform the alignment. M is, for example, greater than 20, 30, or 50. One cycle of sequencing usually can determine the base type at one position in the nucleic acid template. When the sequencing proceeds to cycle M, for example, cycle 20, 50, 80, 100, or 150, crosstalk due to partial overlapping of emission spectra of fluorescent dyes and/or phasing due to asynchronous chemical reactions are generally obvious because of accumulation, superposition, or the like, which is shown as that signals of four types of bases have crosstalk between every two. Sequencing images acquired in cycle M can be used for determining the offset, and thereby the set of sequencing images are aligned.

In some examples, the sequencing images of cycle M of sequencing include a fifth sequencing image, a sixth sequencing image, a seventh sequencing image, and an eighth sequencing image. The fifth sequencing image, the sixth sequencing image, the seventh sequencing image, and the eighth sequencing image each correspond to the same type of nucleotide as the first sequencing image, the second sequencing image, the third sequencing image, and the fourth sequencing image, respectively, in the set of sequencing images.

S14, determining, based on a pixel value or a sub-pixel value at the same basic unit position in one or more aligned sets of sequencing images, the signal intensity at the basic unit position of the images. In practical applications, the brightness of the feature of the corresponding nucleic acid template in the image is always interfered with by various factors, primarily including crosstalk between channels and phasing or prephasing between different cycles of sequencing in the same channel. Therefore, after the sequencing images are aligned and the signal intensity at the same basic unit position is acquired, the signal intensity may further be corrected. The correction includes, but is not limited to, at least one of crosstalk correction, prephasing correction or phasing correction.

In one possible implementation, the process of crosstalk correction includes: a. collecting sequencing data of a reference sample or standard sample including the sequences of different base types with known concentrations; b. removing low-quality bases or sequences in the reference sample or standard sample; c. acquiring interference matrixes or interference parameters between different base types by measuring the reference sample; and d. correcting the signal intensity of the image including the feature of the nucleic acid template based on the interference matrixes or interference parameters. For example, a correction factor may be calculated through a linear model or a non-linear model, and the correction factor is used to correct the signal intensity, thereby reducing the interference effect between different base types.

In one possible implementation, a linear model is established by assuming a linear relationship between the signal intensity of sequencing data and the concentration. For example, a linear regression model or a ridge regression model may be used. Based on the linear model, the interference effect between different base types is estimated, and a correction factor is calculated. The correction factor may be a ratio or difference between the signal intensities and the concentrations of different base types in the reference sample. The correction factor is applied to the image including the feature of the nucleic acid template by multiplying or dividing the signal intensity by the correction factor, thereby reducing the interference effect between different base types.

In another possible implementation, a non-linear model is established to describe the interference effect between different base types. For example, a polynomial regression model or other non-linear models may be used. Based on the nonlinear model, the interference effect between different base types is estimated, and a correction factor is calculated. The correction factor may be a ratio or difference between the signal intensities and the concentrations of different base types in the reference sample. The correction factor is applied to the image including the feature of the nucleic acid template by multiplying or dividing the signal intensity by the correction factor, thereby reducing the interference effect between different base types.

In some examples, the process of phasing correction includes: a. collecting sequencing data of a reference sample or standard sample with known sequences, which include phase information between adjacent bases; b. performing quality control on the reference sample or standard sample, and removing low-quality bases or sequences; c. estimating a phase offset estimation value between adjacent bases through a statistical modeling or graph theory algorithm; for example, the phase offset estimation value may be acquired by calculating the sequence features, the difference in signal intensity, and the like of adjacent bases; and d. correcting the signal intensity of the image including the feature of the nucleic acid template based on the phase offset estimation value. During the correction process, the signal intensity can be adjusted according to the phase offset estimation value to reduce or eliminate the influence of the phase offset on the signal intensity.

In one possible implementation, the sequencing images may be preprocessed to remove the noise background in the images and reduce the interference of the image background on spots corresponding to the reaction signals. The preprocessing on the sequencing image may be a filtering processing.

In one possible implementation, the correction of the signal intensities at the same basic unit position in the sequencing images may include prephasing correction, phasing correction or crosstalk correction. Specifically, after the determination of the pixel value or the sub-pixel value at the same basic unit position in the one or more aligned sets of sequencing images, at least one of S144, S146, and S 148 is performed to determine signal intensities of the four types of bases corresponding to the same basic unit position in one set of sequencing images:

S 144, performing, based on a pixel value or a sub-pixel value of at least one of the three other types of bases than the designated type of base at the same basic unit position in one set of sequencing images, crosstalk correction on a pixel value or a sub-pixel value of the designated type of base.

The three types of bases other than the designated type of base are, for example, any three of the four types of bases (A, T, C, G), and the remaining one type of base is the designated type of base.

In one example, crosstalk correction may be performed on the pixel value or the sub-pixel value of the designated type of base based on the pixel value or the sub-pixel value of one of the three other types of bases than the designated type of base at the same basic unit position in one set of sequencing images by using the formula (I):

$$I_1{}^{'} = I_1 - K_1 \times I_2 \qquad\qquad \text{formula (I)}$$

$I_1{}'$ represents the pixel value or the sub-pixel value of the designated type of base after correction, $I_1$ represents the pixel value or the sub-pixel value of the designated type of base before correction, $I_2$ represents the pixel value or the sub-pixel value of one of three other types of bases than the designated type of base, and $K_1$ represents a correction coefficient.

In another example, crosstalk correction may be performed on the pixel value or the sub-pixel value of the designated type of base based on the pixel values or the sub-pixel values of two of the three other types of bases than the designated type of base at the same basic unit position in one set of sequencing images by using the formula (II):

$$I_1{}^{'} = I_1 - K_1 \times I_2 - K_2 I_3 \qquad\qquad \text{formula (II)}$$

$I_1{}'$ represents the pixel value or the sub-pixel value of the designated type of base after correction, $I_1$ represents the pixel value or the sub-pixel value of the designated type of base before correction, $I_2$ and $I_3$ represent the pixel values or the sub-pixel values of two of the three other types of bases than the designated type of base, and $K_1$ and $K_2$ represent correction coefficients.

In yet another example, crosstalk correction may be performed on the pixel value or the sub-pixel value of the designated type of base based on the pixel values or the sub-pixel values of three of the three other types of bases than the designated type of base at the same basic unit position in one set of sequencing images by using the formula by using the formula (III):

$$I_1{}^{'} = I_1 - K_1 \times I_2 - K_2 I_3 - K_3 I_4 \qquad\qquad \text{formula (III)}$$

$I_1{}'$ represents the pixel value or the sub-pixel value of the designated type of base after correction, $I_1$ represents the pixel value or the sub-pixel value of the designated type of base before correction, $I_2$, $I_3$, and $I_4$ represent the pixel values or the sub-pixel values of the three other types of bases than the designated type of base, and $K_1$, $K_2$, and $K_3$ represent correction coefficients.

S146, performing, based on a pixel value or a sub-pixel value at the same basic unit position in the one set of sequencing images generated in the previous cycle of sequencing, prephasing correction on a pixel value or sub-pixel value at the basic unit position in the set of sequencing images generated in the current cycle of sequencing.

It will be appreciated that prephasing may occur between sequencing images from a plurality of cycles of sequencing, and the sequencing image from a previous cycle may be used to correct the sequencing image from a next cycle. For example, if the pixel value at the basic unit (3,1) of the previous cycle_N-1 is x and the pixel value at the basic unit (3,1) of the next cycle_N is y, the pixel value y may be subjected to prephasing correction based on the pixel value x, thereby reducing the prephasing between the basic unit (3,1) of the previous cycle_N-1 and the basic unit (3,1) of the next cycle_N.

S148, performing, based on a pixel value or a sub-pixel value at the same basic unit position in the one set of sequencing images generated in the next cycle of sequencing, phasing correction on a pixel value or the sub-pixel value of the basic unit position in the set of sequencing images generated in the current cycle of sequencing.

It will be appreciated that phasing may occur between sequencing images from a plurality of cycles of sequencing, and the sequencing image from a next cycle may be used to correct the sequencing image from a previous cycle. For example, if the pixel value at the basic unit (3,1) of the next cycle_N+1 is z and the pixel value at the basic unit (3,1) of the previous cycle_N is x, the pixel value x may be subjected to phasing correction based on the pixel value z, thereby reducing the phasing between the basic unit (3,1) of the next cycle_N+1 and the basic unit (3,1) of the previous cycle_N. In one possible implementation, the signal intensity at the basic unit position may also be normalized to give the final signal intensity at the basic unit position.

S40, detecting, based on the signal intensity at each basic unit position, the type of one or more bases incorporated into the nucleic acid template corresponding to the basic unit position to determine a detected base sequence at each basic unit position.

It will be appreciated that when a base at the corresponding position of the nucleic acid template undergoes base pairing with the reaction substrate to generate a reaction signal, the microscopic imaging system acquires the reaction signal to form an image. The size of the reaction signal in the image is represented as one or more basic units, and the signal intensity at each basic unit position in the image is correlated with the intensity of the reaction signal at the corresponding position of the nucleic acid template corresponding to each basic unit position. Therefore, the signal intensity at each basic unit is capable of characterizing the relative amount of the reaction signal generated during the base pairing between the base at the corresponding position of the nucleic acid template corresponding to each basic unit and the reaction substrate. By detecting the signal intensity at the basic unit in the image, or in combination with the type of the reaction signal (e.g., red light signal, blue light signal), the type of nucleotide incorporated at the corresponding position of the nucleic acid template to which the basic unit position corresponds can be determined. Furthermore, after determining the type of one or more bases incorporated at the corresponding position of the nucleic acid template corresponding to the basic unit position in the image, the detected base sequence at the position of the nucleic acid template can be determined based on the type of the one or more bases incorporated at the corresponding position of the nucleic acid template.

In one possible implementation, one or more reaction signals may be detected at one basic unit position in the image. As such, the one or more bases incorporated at the corresponding position of the nucleic acid template corresponding to the basic unit can be determined based on the signal intensity at the basic unit position. Furthermore, the detected base sequence of the basic unit can be determined by sequentially arranging, in the sequencing order, the one or more base types corresponding to the basic unit.

The embodiments of the present disclosure further provide a specific implementation for determining a detected base based on the signal intensity. The step S40 specifically includes the following steps: S42, determining, based on the signal intensity at the basic unit position, a possibility of each base type incorporated into a corresponding nucleic acid template in the cycle of sequencing.

In the embodiments of the present disclosure, the possibility of incorporating the corresponding type of nucleotide into the corresponding position of the nucleic acid template corresponding to the basic unit position can be determined based on the magnitude of the signal intensity of the reaction signal detected at the basic unit position. For example, in a sequencing platform that performs sequencing-by-synthesis based on surface fluorescence microscopic imaging detection, during each cycle of sequencing by sequencing-by-synthesis, the signal intensity of one basic unit corresponding to the feature of the nucleic acid template in the acquired image may be represented as an array corresponding to four types of bases. For example, the array may be represented as {IntsA, IntsT, IntsG, IntsC}, where IntsA, IntsT, IntsG, and IntsC denote the signals generated from A, T, G, and C, respectively. In a cycle of sequencing, if the signal intensity of one basic unit corresponding to the feature of the nucleic acid template is, for example, {80, 10, 5, 5}, the possibility of incorporating the corresponding type of nucleotide at the corresponding position of the nucleic acid template corresponding to the basic unit position can be determined based on the magnitude of the signal intensity of the reaction signal detected at the basic unit position. Generally, a greater signal intensity corresponds to a higher possibility or probability that the nucleotide type to which the signal intensity points is incorporated into the nucleic acid template. Accordingly, the nucleotide type to which the maximum signal intensity points may be used as the detected base at the basic unit position.

It should be noted that the correspondence between the magnitude of the signal intensity and the possibility of incorporating the corresponding base type in the above-mentioned implementation is provided merely as an example. The embodiments of the present disclosure also support other implementations for determining the possibility of incorporating the corresponding base type according to the magnitude of the signal intensity.

S44, determining, according to a base type with the highest possibility, a detected base at the basic unit position in the cycle of sequencing.

In one possible implementation, based on the determination of the possibility of incorporating a plurality of base types in S42, the base type with the highest possibility may be determined as the detected base at the basic unit position in the cycle of sequencing. Illustratively, in a sequencing platform that performs sequencing-by-synthesis based on surface fluorescence microscopic imaging detection, during each cycle of sequencing by sequencing-by-synthesis, the signal intensity of one basic unit corresponding to the feature of the nucleic acid template in the acquired image may be represented as an array corresponding to four types of bases. For example, the array may be represented as {IntsA, IntsT, IntsG, IntsC}, where IntsA, IntsT, IntsG, and IntsC denote the signals generated from A, T, C, and G, respectively. In a cycle of sequencing, if the signal intensity of one basic unit corresponding to the feature of the nucleic acid template is, for example, {80, 10, 5, 5}, the base type with the highest possibility of being incorporated into the corresponding nucleic acid template in the cycle of sequencing may be determined as A based on the magnitude of the signal intensity of the reaction signal detected at the basic unit position. That is, base A is the detected base at the basic unit position in the cycle of sequencing.

In another possible implementation, a plurality of reaction signals are detected at the basic unit position. Reaction signals with a signal intensity greater than a first preset value are first selected, then the possibility that the corresponding type of base is detected at the basic unit position is determined based on the signal intensity of the selected reaction signals, and the base type with the highest possibility is used as the detected base at the basic unit position in the cycle of sequencing. That is, the base type with the highest possibility and a corresponding signal intensity greater than the first preset value is determined as the detected base at the basic unit position in the cycle of sequencing.

In another possible implementation, a plurality of reaction signals are detected at the basic unit position; the quality score of the corresponding base type is determined based on the signal intensity of each reaction signal, base types with the quality score greater than a second preset value are further selected, and the base type with the highest possibility is selected as the detected base at the basic unit position in the cycle of sequencing. That is, the base type with the highest possibility and a quality score greater than the second preset value is determined as the detected base at the basic unit position in the cycle of sequencing. The quality score may reflect the probability of correct detection of the base. In general, a higher quality score indicates a higher probability of correct detection of the base. S60, clustering, based on a similarity between the detected base sequence at each basic unit position and detected base sequences at surrounding basic unit positions thereof, the detected base sequences or the basic unit positions to determine a portion of the base sequence of the nucleic acid template. Specifically, the detected base sequences at each basic unit position may be clustered, and the similar detected base sequences may be classified as originating from the same nucleic acid template, thereby determining a portion of the base sequence of the nucleic acid template of interest. Alternatively, the same nucleic acid template may be characterized by similar basic unit positions in the image, and the basic unit positions may be clustered to classify similar basic unit positions as corresponding to the same nucleic acid template position.

The detected base sequences may be clustered based on the similarity between the detected base sequences at each basic unit position, or the basic unit positions may be clustered based on the similarity between the detected base sequences at each basic unit position to determine a portion of the base sequence of the nucleic acid template.

In one possible implementation, each of the detected base sequences may be aligned with a reference sequence, and the similarity between the detected base sequences or the similarity between the basic unit positions corresponding to the detected base sequences may be determined based on the alignment result of the detected base sequences. Specifically, step S60 includes the following steps S602 to S606:

S602, aligning detected base sequences at the basic unit position with a reference sequence to acquire an alignment result.

The length of the reference sequence is greater than or equal to the length of the detected base sequence; the reference sequence may be a known gene sequence or one of the detected base sequences selected as the reference. Illustratively, the detected base sequence is at least 4 bp in length. The alignment result includes: success in aligning the detected base sequence to the reference sequence, or failure in aligning the detected base sequence to the reference sequence, i.e., unalignment of the detected base sequence to the reference sequence. The embodiments of the present disclosure further provide a specific implementation for acquiring the above alignment result by using the detected base sequence as the reference sequence. Specifically, the step S602 includes the following steps:

S6021, aligning, by taking any one of the detected base sequences as the reference sequence, other detected base sequences to the reference sequence, and determining, based on the alignment result, a first set of sequences aligned successfully to the reference sequence and a second set of sequences unaligned to the reference sequence.

Specifically, the alignment between the detected base sequence and the reference sequence includes sequentially comparing each base. If the detected base sequence is completely identical to the reference sequence or the detected base sequence is a portion of the reference sequence, the detected base sequence is considered to be aligned successfully to the reference sequence.

Illustratively, assuming that the reference sequence is ATCGTACGATCGTACGATCG with a length equal to or greater than that of each detected base sequence, if the detected base sequence (for example, ATCGTACGATCGTA CGATCG) is identical to the reference sequence or the detected base sequence (for example, ATCGTACGATCGTAC GAT) is a portion of the reference sequence, the detected base sequence is considered to be aligned successfully to the reference sequence.

S6022, aligning, by taking any one of the detected base sequences in the second set of sequences as a new reference sequence, other detected base sequences in the second set of sequences to the new reference sequence, and determining, based on the alignment result, a third set of sequences aligned successfully to the new reference sequence.

It will be appreciated that setting the new reference sequence may allow a further reduction in the number of alignments by excluding the unaligned detected base sequences in the second set of sequences based on the alignment result between each detected base sequence in the second set of sequences and the new reference sequence.

S6023, separating the third set of sequences and repeating S6022 one or more times until the second set of sequences includes 0 detected base sequences, so as to acquire the alignment result.

Separating the third set of sequences from the second set of sequences and repeatedly executing step S6022 may help reduce the number of detected base sequences in the second set of sequences and improve the alignment efficiency.

S604, determining, based on the alignment result, the similarity of the detected base sequences or a similarity of basic unit positions from which the detected base sequences originate, and determining, based on the alignment result, one or more detected base sequences aligned to the same position of the reference sequence as a set of sequences having the same similarity.

Specifically, the detected base sequence may be aligned to a portion of the bases in the reference sequence, or may be aligned to all the bases in the reference sequence. It can be considered that the similarity between the detected base sequence and the reference sequence varies when the detected base sequence is aligned to different positions in the reference sequence. For example, when the reference sequence is ATCGTACGATCGTACGATCG, detected base sequence 1, ATCGTACTTTGC, is aligned to the reference sequence at positions "ATCGTAC", suggesting a 35% similarity between base sequence 1 and the reference sequence; detected base sequence 2, GGGTACGATCGTACG ATCG, is aligned to the reference sequence at positions "TACGATCGTACGATCG", suggesting an 80% similarity between base sequence 2 and the reference sequence.

Illustratively, if different detected base sequences are aligned to the same position in the reference sequence, these detected base sequences can be regarded as having the same similarity to the reference sequence. That is, one or more detected base sequences aligned to the same position in the reference sequence are determined as a set of sequences having the same similarity based on the alignment result. For example, when detected base sequence 1, detected base sequence 3, and detected base sequence 4 are all aligned to positions "ATCGTAC" in the reference sequence, detected base sequence 1, detected base sequence 3, and detected base sequence 4 are determined as a set of sequences having the same similarity. When detected base sequence 2, detected base sequence 5, and detected base sequence 6 are all aligned to positions "TACGATCGTACGATCG" in the reference sequence, detected base sequence 2, detected base sequence 5, and detected base sequence 6 are determined as a set of sequences having the same similarity.

In one possible implementation, binarization processing may be performed for the basic unit positions in the image based on the alignment result to acquire a binary image, and the basic unit positions may be clustered based on the binary image.

S6041, simplifying, based on the alignment result, the image, including: assigning a P1 value to a basic unit position from which a detected base sequence successfully aligned to the reference sequence originates, and assigning a P2 value to a basic unit position from which a detected base sequence unaligned to the reference sequence originates.

For example, if the detected base sequence corresponding to the basic unit position is aligned successfully to the reference sequence, the grayscale value for that basic unit position is set to P1, otherwise the grayscale value for that basic unit position is set to P2. It should be noted that P1 and P2 are significantly distinct grayscale values, allowing accurate characterization of basic unit positions of detected base sequences successfully aligned to the reference sequence and basic unit positions of detected base sequences unaligned to the reference sequence. For example, P1 is the maximum grayscale value, and P2 is the minimum grayscale value.

S6042, clustering and classifying basic unit positions in the simplified image, including: determining all basic unit positions in a range of $k \times k$ basic unit positions with values satisfying a preset distribution as a set of basic unit positions having the same similarity, where k is an odd number greater than 1, and $k \times k$ is greater than 1 pixel.

Specifically, the basic unit positions assigned as P1 in the range of $k \times k$ basic unit positions may be determined as a set of basic unit positions having the same similarity. The $k \times k$ basic unit positions may form a $k \times k$ basic unit matrix in the image.

In one possible implementation, the detected base sequences corresponding to the basic unit positions assigned as P1 in the range of $k \times k$ basic unit positions may originate from the same nucleic acid molecule cluster or from a plurality of different nucleic acid molecule clusters. If the detected base sequences corresponding to the basic unit positions assigned as P1 in the range of $k \times k$ basic unit positions originate from a plurality of different nucleic acid molecule clusters, the basic unit positions corresponding to the different nucleic acid molecule clusters need to be further distinguished.

S606, classifying one or more detected base sequences with a similarity not less than a preset level as originating from one nucleic acid template, or classifying one or more basic unit positions with a similarity not less than a preset level as one nucleic acid template position, so as to acquire a base sequence of each nucleic acid template and a position of each nucleic acid template.

It should be noted that the detected base sequences aligned to the same position in the reference sequence correspond to the same similarity and are classified as a set of detected base sequences having the same similarity. Different sets of detected base sequences correspond to different similarities. A set of detected base sequences with a similarity not less than a preset level may be classified as originating from the same nucleic acid template; alternatively, the basic unit positions corresponding to a set of detected base sequences with a similarity not less than a preset level are classified as the same nucleic acid template position.

In one possible implementation, the method further includes, before step S604: S603, removing a detected base sequence unaligned to the reference sequence, so as to improve the sequencing accuracy.

In another possible implementation, the distance algorithm may also be employed to determine the similarity between the detected base sequence at the basic unit position and the detected base sequences of the surrounding basic units thereof, e.g., using the Hamming distance algorithm for sequences of equal length.

As an example, for two 20-bp DNA sequences, it is assumed that sequence 1 is 5'-ATCGTACGATCGTACG ATCG-3' and sequence 2 is 5'-ATCGTACGTTAGTACGATTA-3'; the Hamming distance between sequence 1 and sequence 2 is calculated by comparing the base difference at the same position of the two sequences position by position and counting the number of unmatched positions. In this example, the bases at 4 positions are unmatched, so the Hamming distance between the two sequences is 4.

After the Hamming distance is calculated, the score describing the similarity between the two sequences may be calculated using the following formula: score = sequence length - Hamming distance. For the above example, the calculated score is 16. A higher score indicates a higher similarity between the two sequences. When it is necessary to determine the similarity between the detected base sequence of the basic unit and the detected base sequences of the surrounding basic units, the detected base sequence of the basic unit may be individually scored against each of the detected base sequences of the surrounding basic units thereof in accordance with the aforementioned method, and the resulting scores may be summed to determine the similarity between the detected base sequence of the basic unit and the detected base sequences of the surrounding basic units.

In some examples, the clustering result of the foregoing steps includes one or more sets of sequences (i.e., base sequences) demonstrating high similarity, where each set of sequences may originate from the same nucleic acid template. Alternatively, by clustering the basic unit positions in the foregoing clustering process, a region of the same nucleic acid template containing highly similar base sequences can be acquired. The aforementioned nucleic acid template region may be a region where a plurality of identical polynucleotide molecules or a nucleic acid molecule cluster formed by the amplification of the nucleic acid template are located. The sequences demonstrating high similarity described above may originate from a plurality of identical polynucleotide molecules or a nucleic acid molecule cluster formed by the same nucleic acid template. Adjacent sequences demonstrating high similarity are merged to yield a merged base sequence (uniqRead), which corresponds to a portion of the base sequence of the nucleic acid template.

Illustratively, for each set of sequences demonstrating high similarity, multiple sequence alignment tools (such as MAFFT and Clustal Omega) are used to align the sequences with high similarity in the set, so as to determine their similar regions. Then, based on the result of multiple sequence alignments, the adjacent sequences demonstrating high similarity in the set are merged to yield the merged base sequence. Considering that the merged base sequence may contain inaccuracies due to sequencing errors or clustering errors, the potential errors in the merged base sequence may be verified and corrected by aligning the merged base sequence to a reference genome.

In one possible implementation, for the detected base sequence of a certain basic unit position, the basic unit position may also be scored based on the differences between the detected base sequence and the detected base sequences at the surrounding basic unit positions of the basic unit position, such that the basic unit positions, or the detected base sequences, are clustered based on the score of the basic unit position.

Specifically, S60 includes: determining base differences between the detected base sequence at the basic unit position and the detected base sequences at surrounding $(k \times k - 1)$ basic unit positions thereof, determining, based on all the base differences, a score of the basic unit position, and performing, based on the score of the basic unit position, the clustering,

where k is an odd number greater than 1; the basic unit and the surrounding $(k \times k - 1)$ basic units thereof constitute a $K \times K$ pixel matrix in the sequencing image with the basic unit located at the center of the $K \times K$ pixel matrix.

In one possible implementation, performing, based on the score of the basic unit position, the clustering includes: if the score of the basic unit position is greater than a third preset value, clustering the detected base sequence at the basic unit position and the detected base sequences at the surrounding $(k \times k - 1)$ basic unit positions thereof, or, clustering the basic unit position and the surrounding $(k \times k - 1)$ basic unit positions thereof.

In some examples, by performing an exclusive OR operation on the detected base sequence of each basic unit and the detected base sequences of the surrounding $(k \times k - 1)$ basic units thereof, the base differences between the detected base sequence of each basic unit and the detected base sequences of the surrounding $(k \times k - 1)$ basic units thereof can be determined. In addition, the sum of all the difference values is calculated, and the score of each basic unit is derived based on the difference between a fixed threshold and the sum. The fixed threshold is used to characterize the sum of the lengths of the detected base sequences of the surrounding (k × k - 1) basic units in the case that the similarity between the detected base sequence of each basic unit and the detected base sequences of the surrounding (k × k - 1) basic units thereof is 100%.

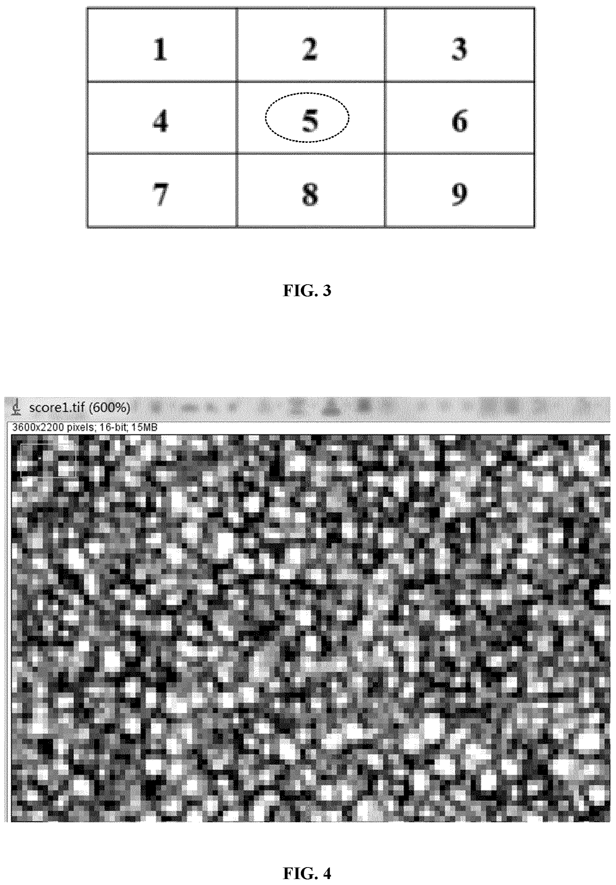

In some examples, referring to FIG. 3, it is assumed that the pixel matrix is 3 × 3, containing a total of 9 pixel positions numbered 1-9; base calling is performed on the nucleic acid templates at each of pixel positions 1-9 for 8 cycles. As a result, each pixel position yields a read (base sequence) with a length of 8 bp (referring to the length of the sequence; the length of DNA is expressed in bp (i.e., base pair)). These reads are denoted as read1 to read9, respectively. For example, each read records only the base type information, without recording the Q30 value and grayscale value (intensity) of the bases on each read. A score is calculated for pixel position 5 to represent the similarity between the detected base sequence at that pixel position and the detected base sequences at the surrounding pixel positions thereof. The score for the pixel position 5 (i.e., score5) is calculated as follows:

$$\text{Score5} = 64 - (\text{read5}^\wedge\text{read1} + \text{read5}^\wedge\text{read2} + \text{read5}^\wedge\text{read3} + \text{read5}^\wedge\text{read4}$$

$$+ \text{read5}^\wedge\text{read6} + \text{read5}^\wedge\text{read7} + \text{read5}^\wedge\text{read8})$$

The exclusive OR calculation between the reads is to compare the bases of the two reads at the same position. If the bases are the same, 0 is added, and if the bases are different, 1 is added. Thus, the result of a single exclusive OR operation between two 8-bp reads, readN ^ readM, falls within the value range [0,8], meaning at most 8 identical bases and at least 0 identical bases. The final score has a value range of [0,64]. A higher score indicates a higher similarity between the detected base sequence at the pixel position and the detected base sequences at the surrounding pixel positions thereof, and suggests that these base sequences are more likely to originate from the same nucleic acid template.

The score for each pixel position is calculated to form a score map, as shown in FIG. 4, where the position of the local brightest spot corresponds to the nucleic acid template.

On the score map shown in FIG. 4, local optimum search may be used to find the maximum value within a 3 × 3 range, i.e., the feature position of the corresponding nucleic acid template. If a plurality of maximum values 64 are encountered, the center is selected, or the positions are bounded by 3 × 3 and are marked as a plurality of adjacent nucleic acid templates, which may suggest the presence of one large nucleic acid template.

On the basis of the above, longer cycles may also be used for determining the nucleic acid template. For example, when the similarity is obtained by performing exclusive OR operations on 20-bp reads, more reads can be found in high-density data, thus achieving a higher throughput; alternatively, data related to Q30 and/or intensity may be recorded in the read, and Q30 and/or intensity may be used to assist in confirming the position of the nucleic acid template. In areas without apparent local optimum, this additional information may be used to find scattered nucleic acid templates that appear as 1-2 pixels or smaller in size in the image, while the throughput can be improved.

In some examples, taking a basic unit representing a pixel as an example, each pixel position and the surrounding (k × k - 1) pixel positions thereof constitute a K × K pixel matrix in the sequencing image.

In another possible implementation, the basic unit positions, or the detected base sequences, may be clustered based on a variation in the score of the basic unit position. Specifically, S60 includes: determining base differences between the detected base sequence at each basic unit position and the detected base sequences at surrounding (k × k - 1) basic unit positions thereof, and determining, based on the base differences, a score of each basic unit position; and performing, based on the variation in the score of each basic unit position, the clustering.

Each basic unit and the surrounding (k × k - 1) basic units thereof constitute a K × K pixel matrix in the sequencing image.

Specifically, performing, based on a variation in the score of each basic unit position, the clustering includes: if a maximum is present in the scores of the basic unit position and the surrounding (k × k - 1) basic unit positions thereof, clustering the basic unit position and the surrounding (k × k - 1) basic unit positions thereof, or, clustering the detected base sequence at the basic unit position and the detected base sequences at the surrounding (k × k - 1) basic unit positions.

Illustratively, taking a basic unit representing a pixel as an example, the pixel matrix is 3 × 3, containing a total of 9 pixel positions numbered 1-9. The scores of the detected base sequences read1 to read9 corresponding to pixels 1-9 can be acquired based on the method described above, and are denoted as score1 to score9, respectively. If the maximum value is found in score1 to score9, either read1 to read9 or pixel 1 to pixel 9 may be clustered.

The method for determining the base sequence of the nucleic acid template according to the present disclosure includes: performing base calling on each basic unit of the image, determining the detected base sequence of each basic unit, and clustering the detected base sequences or the basic unit positions based on the detected base sequence of each basic unit. Since the nucleic acid molecule clusters are formed by the amplification of the nucleic acid template, the base sequences originating from the same nucleic acid molecule cluster are likely to be similar. Therefore, all the detected base sequences or all the basic units are clustered based on the detected base sequence of each basic unit, and a portion of the base sequence of the nucleic acid template can be determined based on the clustering result. Compared with the method for performing base calling by constructing a template in the prior art, the method provided in the embodiments of the present disclosure enables accurate determination of the base sequence of the nucleic acid template. This avoids the target signal detection loss or omission of certain nucleic acid templates caused by weak reaction signals (relatively weak target signals), low sensitivity of the used signal detection or acquisition mode, and/or other reasons, thereby improving the sequencing accuracy and sequencing throughput.

In one embodiment of the present disclosure, the method further includes: aligning the detected base sequences of the basic units with the reference sequence, and removing the base sequences that do not match the reference sequence from the detected base sequences of the basic units.

In some examples, taking a basic unit representing a pixel as an example, the detected base sequence at each pixel position is aligned with the reference sequence set using a sequence alignment algorithm (e.g., Basic Local Alignment Search Tool (BLAST), Bowtie, and Burrows-Wheeler Aligner (BWA)) to generate an alignment result, the detected base sequences that do not match the reference sequence set are removed based on the alignment result, and the matched detected base sequences are retained as the final result. It should be noted that the process of aligning and removing unmatched base sequences may require parameter setting and filtering based on actual conditions to ensure the accuracy and reliability. In addition, the alignment result may also be further analyzed and interpreted according to experimental requirements and specific analysis objectives.

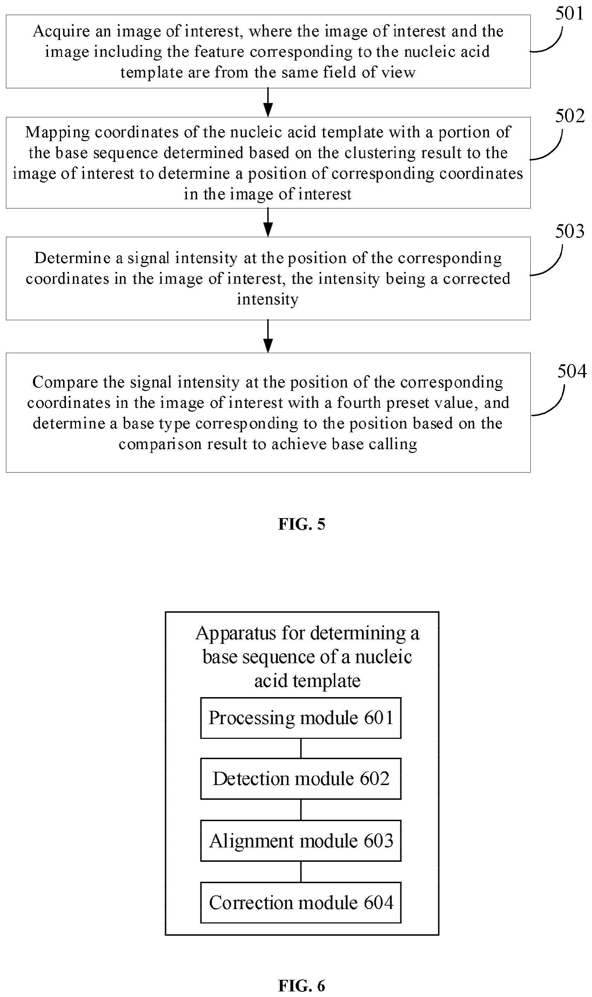

In one embodiment of the present disclosure, referring to FIG. 5, the method provided in the embodiments of the present disclosure further includes the following steps:

501, acquiring an image of interest, where the image of interest and the image including the feature corresponding to the nucleic acid template are from the same field of view.

It will be appreciated that the image of interest and the image containing the features of the corresponding nucleic acid template are acquired in the same area, but possibly at different points in time or using different imaging techniques (e.g., different fluorescence labels or imaging conditions). 502, mapping coordinates of the nucleic acid template with a portion of the base sequence determined based on the clustering result to the image of interest to determine a position of corresponding coordinates in the image of interest.

In some examples, the coordinate mapping is to establish a mapping relationship between the original image, such as a set corresponding to the nucleic acid templates with a portion of the base sequence determined based on the clustering result, and the target image, such as the image of interest. The mapping relationship here includes determining the coordinate position of any nucleic acid template of the original image in the image of interest after mapping.

The method for determining the coordinates and the method for implementing the coordinate mapping are not limited in this embodiment. Coordinate mapping can be implemented, for example, by a remap function in Opencv.

503, determining a signal intensity at the position of the corresponding coordinates in the image of interest, the intensity being a corrected intensity.

In some examples, the signal intensity is measured at the position of the corresponding coordinates in the image of interest.

The signal intensity at the position of the corresponding coordinates in the image of interest is an array containing four values (four-dimensional data) corresponding to signal intensity of four nucleotides/bases at that position, which, for example, can be expressed as {IntsA, IntsT, IntsG, IntsC}, IntsA, IntsT, IntsG and IntsC representing the signal intensity values of bases A, T, G and C, respectively. After correction, in general, IntsA, IntsT, IntsG, and IntsC have the same baseline, and the maximum value (max) in the array may be compared to a first preset value. If the maximum value is greater than or equal to the first preset value, it can be determined that the base type corresponding to the position in the image is the base corresponding to the maximum value, that is, the base at a corresponding position on the corresponding nucleic acid molecule is called to be the base corresponding to the maximum value; if the maximum value (max) in the array is less than the first preset value, it can be determined that the base type corresponding to the position in the image cannot be accurately called, and the base at the position of the corresponding nucleic acid molecule can be marked as N or that position can be left vacant, N being any one of A, T, G, and C; in some examples, the reads containing N or vacant positions after base calling may be further processed (for example, the base type represented by the N or vacant positions in the reads can be further inferred based on information of other reads, e.g., adjacent reads, or the reads are partially filtered) to improve utilization or quality of the resulting data.

In some examples, the values in {IntsA, IntsT, IntsG, IntsC} are processed, e.g., normalized, values. In some embodiments, the intensity correction includes crosstalk correction, prephasing correction and/or phasing correction.

In some examples, the intensity correction includes crosstalk correction, which is performed based on at least one of the images from the same cycle of sequencing and the same field of view and corresponding to different types of nucleotides/bases.

Crosstalk correction is favorable for accurate base calling. In some examples, image Xi and the image of interest are from the same cycle of sequencing, the image Xi and the image of interest correspond to the same field of view, the image of interest is subjected to crosstalk by signals of a nucleotide corresponding to image Xi, and the crosstalk correction of the image of interest includes: fitting signals of positions of a plurality of corresponding coordinates in a specific area of the image of interest to obtain a fitting result, and correcting the signals/signal intensity of the positions of the corresponding coordinates in the image of interest based on the fitting result. In this way, the signal crosstalk from the base corresponding to image Xi in the image of interest can be eliminated, such that the signals in the image of interest only correspond to one base as much as possible, which is favorable for accurate base calling and accurate determination of the nucleotide sequence.

Unless otherwise stated, "AC correction" or "A-> C" or "A-C" represents correcting the crosstalk of the A signal at positions of corresponding coordinates of the C image (i.e., correcting the crosstalk of the A signal to the C signal); similarly, "TA correction" or "T->A" represents correcting the crosstalk of the T signal at positions of corresponding coordinates of the A image (i.e., correcting the crosstalk of the T signal to the A signal), and "CG correction" or "C->G" represents correcting the crosstalk of the C signal at positions of corresponding coordinates of the G image (i.e., correcting the crosstalk of the C signal to the G signal), and so on.

In some other examples, the intensity correction includes phasing correction, which is performed based on at least one of the images from adjacent cycles of sequencing and corresponding to the same type of nucleotide.

Furthermore, in one example, image Yj and the image of interest are from two adjacent cycles of sequencing (e.g., image Yj is from cycle 31 of sequencing, and the image of interest is from cycle 30 of sequencing), image Yj and the image of interest correspond to the same field of view, image Yj and the image of interest correspond to the same type of nucleotide/base (e.g., A), and the phasing correction includes: fitting signals of positions of a plurality of corresponding coordinates in the image of interest to acquire a fitting result, and correcting the signals of the positions of the corresponding coordinates in the image of interest based on the fitting result.

504, comparing the signal intensity at the position of the corresponding coordinates in the image of interest with a fourth preset value, and determining a base type corresponding to the position based on the comparison result to achieve base calling.

It will be appreciated that the various units described in the following apparatus correspond to the respective steps in the method described with reference to FIG. 2. As such, the operations and features described above for the method are also applicable to the classification information determination apparatus and the units included therein, which will not be recited here. The apparatus may be implemented in a browser or other security applications of a computer device in advance, or may also be loaded into the browser or other security applications of the computer device through methods such as downloading. The corresponding units in the apparatus may cooperate with the units in the computer device to implement the solutions according to the embodiments of the present disclosure. Although several modules or units have been mentioned in the above detailed description, such a division is not mandatory. Indeed, the features and functions of two or more modules or units described above may be embodied in one module or unit according to the embodiments of the present disclosure. Conversely, the features and functions of one module or unit described above may be further divided and embodied in a plurality of modules or units.

It should be noted that details that are not disclosed in the apparatus according to the embodiments of the present disclosure refer to the details disclosed in the above-mentioned embodiments of the present disclosure, which will not be recited here.

FIG. 6 is a schematic block diagram of an apparatus for determining a base sequence of a nucleic acid template according to one embodiment of the present disclosure.

As shown in FIG. 6, the apparatus for determining the base sequence of the nucleic acid template includes: a processing module 601 and a detection module 602.

The processing module 601 is configured for processing an image including a feature corresponding to the nucleic acid template, including: determining a signal intensity at each basic unit in the image, where the image includes a plurality of basic units, the size of the feature corresponding to the nucleic acid template in the image is represented as one or more basic units, and the size of one basic unit is less than or equal to the size of one pixel of the image.

The detection module 602 is configured for detecting, based on the signal intensity at each basic unit position, the type of one or more bases incorporated into the nucleic acid template corresponding to the basic unit position to determine a detected base sequence at each basic unit position.

The detection module 602 is further configured for clustering, based on a similarity between the detected base sequence at each basic unit position and detected base sequences at surrounding basic unit positions thereof, the detected base sequences or the basic unit positions to determine a portion of the base sequence of the nucleic acid template.

In some embodiments, the nucleic acid template is a single-molecule polynucleotide.

In some embodiments, one nucleic acid template includes a plurality of polynucleotide molecules having identical sequences.

In some embodiments, the size of one basic unit is 1 pixel, 0.8 pixels, 0.5 pixels, 0.25 pixels, 0.2 pixels, or 0.1 pixels.

In some embodiments, the apparatus further includes an alignment module 603. The alignment module 603 is configured for performing, by using a sequencing-by-synthesis method based on surface multi-channel fluorescence microscopic imaging, one or more cycles of sequencing on a plurality of the nucleic acid templates connected to a chip surface to generate one or more corresponding sets of sequencing images, where one set of sequencing images generated by each cycle of sequencing includes a plurality of sequencing images corresponding to four types of bases incorporated into the nucleic acid templates, and the sequencing images have identical resolutions and sizes;

> aligning the one or more sets of sequencing images; and
> determining, based on a pixel value or a sub-pixel value at the same basic unit position in one or more aligned sets of sequencing images, the signal intensity at the basic unit position of the images.

In some embodiments, the signal intensity at the basic unit position in the images is a corrected signal intensity, and the correction includes at least one of crosstalk correction or phasing correction.

In some embodiments, the alignment module 603 is specifically configured for: S142, determining the pixel value or the sub-pixel value at the same basic unit position in the one or more aligned sets of sequencing images, and performing at least one of S144, S146, and S148 to determine signal intensities of the four types of bases corresponding to the same basic unit position in one set of sequencing images;

S144, performing, based on a pixel value or a sub-pixel value of at least one of the three other types of bases than the designated type of base at the same basic unit position in one set of sequencing images, crosstalk correction on a pixel value or a sub-pixel value of the designated type of base; S146, performing, based on a pixel value or a sub-pixel value at the same basic unit position in the one set of sequencing images generated in the previous cycle of sequencing, prephasing correction on a pixel value or sub-pixel value at the basic unit position in the set of sequencing images generated in the current cycle of sequencing.

S148, performing, based on a pixel value or a sub-pixel value at the same basic unit position in the one set of sequencing images generated in the next cycle of sequencing, phasing correction on a pixel value or the sub-pixel value of the basic unit position in the set of sequencing images generated in the current cycle of sequencing.

In some embodiments, the signal intensity at the basic unit position in the images is a normalized signal intensity.

In some embodiments, the detection module 602 is specifically configured for: S42, determining, based on the signal intensity at the basic unit position, a possibility of each base type incorporated into a corresponding nucleic acid template in the cycle of sequencing; and

S44, determining a base type with the highest possibility as a detected base at the basic unit position in the cycle of sequencing.

In some embodiments, the detection module 602 is specifically configured for: determining the base type with the highest possibility and a corresponding signal intensity greater than a first preset value as the detected base at the basic unit position in the cycle of sequencing.

In some embodiments, the detection module 602 is specifically configured for: determining the base type with the highest possibility and a quality score greater than a second preset value as the detected base at the basic unit position in the cycle of sequencing, where the quality score is determined via the signal intensity at the basic unit position.

In some embodiments, the detection module 602 is specifically configured for: S602, aligning detected base sequences at the basic unit position with a reference sequence to acquire an alignment result, where the length of the reference sequence is greater than or equal to the length of the detected base sequence;

> S604, determining, based on the alignment result, the similarity of the detected base sequences or a similarity of basic unit positions from which the detected base sequences originate; and
> S606, classifying one or more detected base sequences with a similarity not less than a preset level as one nucleic acid template, or classifying one or more basic unit positions with a similarity not less than a preset level as one nucleic acid template position, so as to acquire a base sequence of each nucleic acid template and a position of each nucleic acid template.

In some embodiments, the detection module 602 is further configured for: removing a detected base sequence unaligned to the reference sequence.

In some embodiments, the detection module 602 is specifically configured for: determining, based on the alignment result, one or more detected base sequences aligned to the same position of the reference sequence as a set of sequences having the same similarity.

In some embodiments, the detection module 602 is specifically configured for: S6041, simplifying, based on the alignment result, the image, including: assigning a P1 value to a basic unit position from which a detected base sequence successfully aligned to the reference sequence originates, and assigning a P2 value to a basic unit position from which a detected base sequence unaligned to the reference sequence originates; and

S6042, clustering and classifying basic unit positions in the simplified image, including: determining all basic unit positions in a range of k × k basic unit positions with values satisfying a preset distribution as a set of basic unit positions having the same similarity, where k is an odd number greater than 1, and k × k is greater than 1 pixel.

In some embodiments, the detection module 602 is specifically configured for: S6021, aligning, by taking any one of the detected base sequences as the reference sequence, other detected base sequences to the reference sequence, and determining, based on the alignment result, a first set of sequences aligned to the reference sequence and a second set of sequences unaligned to the reference sequence;

S6022, aligning, by taking any one of the detected base sequences in the second set of sequences as the reference sequence, other detected base sequences in the second set of sequences to the reference sequence, and determining, based on the alignment result, a third set of sequences aligned to the reference sequence; and
S6023, separating the third set of sequences and repeating S6022 one or more times until the second set of sequences includes 0 detected base sequences, so as to acquire the alignment result.

In some embodiments, the detection module 602 is specifically configured for: determining base differences between the detected base sequence at the basic unit position and the detected base sequences at surrounding (k × k - 1) basic unit positions thereof, and determining, based on all the base differences, a score of the basic unit position, where k is an odd number greater than 1; and performing, based on the score of the basic unit position, the clustering.

In some embodiments, the basic unit and the surrounding (k × k - 1) basic units thereof constitute a K × K pixel matrix in the sequencing image with the basic unit located at the center of the K × K pixel matrix.

In some embodiments, the detection module 602 performs the clustering based on the scores of the basic unit positions, including:
if the score of the basic unit position is greater than a third preset value, clustering the detected base sequence at the basic unit position and the detected base sequences at the surrounding (k × k - 1) basic unit positions thereof, or, clustering the basic unit position and the surrounding (k × k - 1) basic unit positions thereof.

In some embodiments, the detection module 602 is specifically configured for: determining base differences between the detected base sequence at each basic unit position and the detected base sequences at surrounding (k × k - 1) basic unit positions thereof, and determining, based on the base differences, a score of each basic unit position; and performing, based on a variation in the score of each basic unit position, the clustering.

In some embodiments, each basic unit and the surrounding (k × k - 1) basic units thereof constitute a K × K pixel matrix in the sequencing image.

In some embodiments, the detection module 602 performs the clustering based on a variation in the score of each basic unit position, including:
if a maximum is present in the scores of the basic unit position and the surrounding (k × k - 1) basic unit positions thereof, clustering the basic unit position and the surrounding (k × k - 1) basic unit positions thereof, or, clustering the detected base sequence at the basic unit position and the detected base sequences at the surrounding (k × k - 1) basic unit positions.

In some embodiments, the apparatus further includes a correction module 604 configured for: acquiring an image of interest, where the image of interest and the image including the feature corresponding to the nucleic acid template are from the same field of view;

mapping coordinates of the nucleic acid template with a portion of the base sequence determined based on the clustering result to the image of interest to determine a position of corresponding coordinates in the image of interest;
determining a signal intensity at the position of the corresponding coordinates in the image of interest, the signal intensity being a corrected signal intensity; and
comparing the signal intensity at the position of the corresponding coordinates in the image of interest with a fourth preset value, and determining a base type corresponding to the position based on the comparison result to achieve base calling.

It will be appreciated that the various units described in the apparatus for determining the base sequence of the nucleic acid template correspond to the respective steps in the method described with reference to FIG. 1. As such, the operations and features described above for the method are also applicable to the apparatus and the units included therein, which will not be recited here. The apparatus may be implemented in a browser or other security applications of a computer device in advance, or may also be loaded into the browser or other security applications of the computer device through methods such as downloading. The corresponding units in the apparatus may cooperate with the units in the computer device to implement the solutions according to the embodiments of the present disclosure.

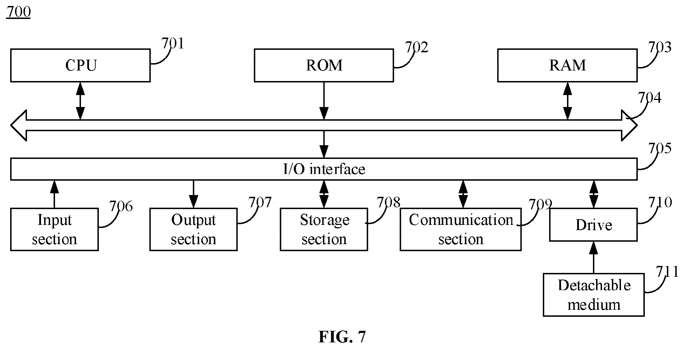

Referring now to FIG. 7, FIG. 7 shows a structural schematic diagram of a computer device suitable for implementing the embodiments of the present disclosure. As shown in FIG. 7, a computer system 700 includes a central processing unit (CPU) 701 that can execute various appropriate actions and processes based on a program stored in a read-only memory (ROM) 702 or a program loaded from a storage section 708 into a random access memory (RAM) 703.

In the RAM 703, various programs and data necessary for operation instructions of the system are also stored. The CPU 701, the ROM 702, and the RAM 703 are connected to each other via a bus 704. An input/output (I/O) interface 705 is also connected to the bus 704.

The following components are connected to the I/O interface 705: an input section 706 including a keyboard, a mouse, and the like; an output section 707 including components such as a cathode ray tube (CRT) and a liquid crystal display (LCD), a speaker, and the like; a storage section 708 including a hard disk and the like; and a communication section 709 including a network interface card such as a LAN card, a modem, and the like. The communication section 709 executes communication processing via a network such as the Internet. A drive 710 is also connected to the I/O interface 705 as needed. A detachable medium 711 such as a magnetic disk, an optical disk, a magneto-optical disk, a semiconductor memory, and the like is mounted on the drive 710 as needed, such that the computer program read out therefrom is mounted in the storage section 708 as required.

In particular, the processes described above with reference to the flowchart of FIG. 1 may be implemented as a computer software program according to the embodiments of the present disclosure. For example, the embodiments of the present disclosure include a computer program product including a computer program embodied on a computer-readable medium. The computer program includes program codes for executing the method illustrated in the flowchart. In such embodiments, the computer program includes program codes for executing the method illustrated in the flowchart. In such embodiments, the computer program may be downloaded from the network via the communication section 709 and installed, and/or installed from the detachable medium 711. The computer program, when executed by the central processing unit (CPU) 701, executes the above functions defined in the system of the present disclosure.

It should be noted that the computer-readable medium shown in the present disclosure may be a computer-readable signal medium, a computer-readable storage medium, or any combination of the two. The computer-readable storage medium may be, for example, but not limited to, an electronic, magnetic, optical, electromagnetic, infrared, or semiconductor system, apparatus, or device, or any combination thereof. More specific examples of the computer-readable storage medium may include, but are not limited to: an electrical connection having one or more wires, a portable computer disk, a hard disk, a random access memory (RAM), a read-only memory (ROM), an erasable programmable read-only memory (EPROM or flash memory), an optical fiber, a portable compact disc read-only memory (CD-ROM), an optical storage device, a magnetic storage device, or any suitable combination thereof. In the present disclosure, the computer-readable storage medium may be any tangible medium containing or storing a program for use by or in conjunction with an instruction execution system, apparatus, or device. Moreover, in the present disclosure, the computer-readable signal medium may include data signals propagated in baseband or as part of a carrier wave that carries computer-readable program codes. These propagated data signals may be in various forms, including but not limited to, electromagnetic signals, optical signals, or any suitable combination thereof. The computer-readable signal medium may also be any computer-readable medium other than the computer-readable storage medium. The computer-readable storage medium may send, propagate, or transmit a program for use by or use in combination with an instruction execution system, apparatus, or device. The program codes contained in the computer-readable medium may be transmitted using any suitable medium, including but not limited to, wireless means, wires, optical cables, RF, or any suitable combination thereof.

The flowcharts and block diagrams in the drawings illustrate the architecture, functions, and operation instructions of possible implementations of the system, method, and computer program product according to various embodiments of the present disclosure. In this regard, each block in the flowcharts or block diagrams may represent a module, a program segment, or a portion of a code, including one or more executable instructions for implementing the specified logical functions. It should also be noted that in some alternative implementations, the functions indicated in the blocks may be implemented in an order different from that indicated in the drawings. For example, two blocks shown in succession may, in fact, be executed substantially concurrently, or the blocks may sometimes be executed in a reversed order, depending upon the functions involved. Also, it should be noted that each block in the block diagrams and/or flowcharts, as well as the combination of blocks in the block diagrams and/or flowcharts, may be executed using a specialized hardware-based system performing the specified functions or operation instructions, or using a combination of specialized hardware and computer instructions.

The units or modules described in the embodiments of the present disclosure may be implemented through software or hardware. The described units or modules may also be provided in a processor. For example, this may be described as: a processor includes a semantic extraction unit, a weight assignment unit, and a determination unit. The names of these units or modules, in some cases, do not limit the units or modules themselves.

As another aspect, the present disclosure further provides a computer-readable storage medium. The computer-readable storage medium may be included in the computer device described in the above embodiments, or may exist separately without being assembled into the computer device. The computer-readable storage medium stores one or more programs which, when executed by one or more processors, execute the methods according to the present disclosure. For example, the steps of the method as shown in FIG. 1 may be executed.

The embodiments of the present disclosure provide a computer program product, including an instruction, where the instruction, when operated, enables the execution of the method as described in the embodiments of the present disclosure. For example, the steps of the method as shown in FIG. 1 may be executed.

The foregoing description is merely preferred embodiments of the present disclosure and is illustrative of the principles of the technology employed. It will be appreciated by those skilled in the art that the scope of the present disclosure is not limited to technical solutions formed by specific combinations of the above technical features, but should also encompass other technical solutions formed by any combination of the above technical features or their equivalent features without departing from the spirit of the present disclosure. For example, technical solutions are formed by substituting the above features with technical features having similar functions as disclosed (but not limited to) in the present disclosure.

**Claims**

1. A method for determining a base sequence of a nucleic acid template, comprising:

   S20, processing an image comprising a feature corresponding to the nucleic acid template, comprising: determining a signal intensity at each basic unit position in the image, wherein the image comprises a plurality of basic units, the size of the feature corresponding to the nucleic acid template in the image is represented as one or more basic units, and the size of one basic unit is less than or equal to the size of one pixel of the image;

   S40, detecting, based on the signal intensity at each basic unit position, the type of one or more bases incorporated into the nucleic acid template corresponding to the basic unit position to determine a detected base sequence at each basic unit position; and

   S60, clustering, based on a similarity between the detected base sequence at each basic unit position and detected base sequences at surrounding basic unit positions thereof, the detected base sequences or the basic unit positions to determine a portion of the base sequence of the nucleic acid template.

2. The method according to any one of claim 1, wherein

   the size of one basic unit is 1 pixel, 0.8 pixels, 0.5 pixels, 0.25 pixels, 0.2 pixels, or 0.1 pixels; optionally, the nucleic acid template is a single-molecule polynucleotide;

   optionally, one nucleic acid template comprises a plurality of polynucleotide molecules having identical sequences.

3. The method according to any one of claim 1 or 2, further comprising:

   S10, performing, by using a sequencing-by-synthesis method based on surface multi-channel fluorescence microscopic imaging, one or more cycles of sequencing on a plurality of the nucleic acid templates connected to a chip surface to generate one or more corresponding sets of sequencing images, wherein one set of sequencing images generated by each cycle of sequencing comprises a plurality of sequencing images corresponding to four types of bases incorporated into the nucleic acid templates, and the sequencing images have identical resolutions and sizes;

   S12, aligning the one or more sets of sequencing images; and

   S14, determining, based on a pixel value or a sub-pixel value at the same basic unit position in one or more aligned sets of sequencing images, the signal intensity at the basic unit position of the images;

   optionally, the signal intensity at the basic unit position in the images is a corrected signal intensity, and the correction comprises at least one of crosstalk correction, prephasing correction or phasing correction.

4. The method according to claim 3, wherein S14 comprises:

   S142, determining the pixel value or the sub-pixel value at the same basic unit position in the one or more aligned sets of sequencing images, and performing at least one of S144, S146, and S148 to determine signal intensities of the four types of bases corresponding to the same basic unit position in one set of sequencing images;

   S144, performing, based on a pixel value or a sub-pixel value of at least one of the three other types of bases than the designated type of base at the same basic unit position in one set of sequencing images, crosstalk correction on a pixel value or a sub-pixel value of the designated type of base;

   S146, performing, based on a pixel value or a sub-pixel value at the same basic unit position in the one set of sequencing images generated in the previous cycle of sequencing, prephasing correction on a pixel value or sub-pixel value at the basic unit position in the set of sequencing images generated in the current cycle of sequencing;

and

S148, performing, based on a pixel value or a sub-pixel value at the same basic unit position in the one set of sequencing images generated in the next cycle of sequencing, phasing correction on a pixel value or the sub-pixel value of the basic unit position in the set of sequencing images generated in the current cycle of sequencing; optionally, the signal intensity at the basic unit position in the images is a normalized signal intensity.

5. The method according to any one of claim 3 or 4, wherein S40 comprises:

S42, determining, based on the signal intensity at the basic unit position, a possibility of each base type incorporated into a corresponding nucleic acid template in the cycle of sequencing; and
S44, determining a base type with the highest possibility as a detected base at the basic unit position in the cycle of sequencing;
optionally, S44 comprises: determining the base type with the highest possibility and a corresponding signal intensity greater than a first preset value as the detected base at the basic unit position in the cycle of sequencing;
optionally, S44 comprises: determining the base type with the highest possibility and a quality score greater than a second preset value as the detected base at the basic unit position in the cycle of sequencing, wherein the quality score is determined via the signal intensity at the basic unit position.

6. The method according to any one of claims 1-5, wherein S60 comprises:

S602, aligning detected base sequences at the basic unit position with a reference sequence to acquire an alignment result, wherein the length of the reference sequence is greater than or equal to the length of the detected base sequence;
S604, determining, based on the alignment result, the similarity of the detected base sequences or a similarity of basic unit positions from which the detected base sequences originate; and
S606, classifying one or more detected base sequences with a similarity not less than a preset level as originating from one nucleic acid template, or classifying one or more basic unit positions with a similarity not less than a preset level as one nucleic acid template position, so as to acquire a base sequence of each nucleic acid template and a position of each nucleic acid template;
optionally, further comprising:
S603, removing a detected base sequence unaligned to the reference sequence.

7. The method according to claim 6, wherein S604 comprises:
determining, based on the alignment result, one or more detected base sequences aligned successfully to the same position of the reference sequence as a set of sequences having the same similarity.

8. The method according to claim 6, wherein S604 comprises:

S6041, simplifying, based on the alignment result, the image, including: assigning a P1 value to a basic unit position from which a detected base sequence successfully aligned to the reference sequence originates, and assigning a P2 value to a basic unit position from which a detected base sequence unaligned to the reference sequence originates; and
S6042, clustering and classifying basic unit positions in the simplified image, comprising: determining all basic unit positions in a range of k × k basic unit positions with values satisfying a preset distribution as a set of basic unit positions having the same similarity, wherein k is an odd number greater than 1, and k × k is greater than 1 pixel.

9. The method according to claim 6, wherein S602 comprises:

S6021, aligning, by taking any one of the detected base sequences as the reference sequence, other detected base sequences to the reference sequence, and determining, based on the alignment result, a first set of sequences aligned successfully to the reference sequence and a second set of sequences unaligned to the reference sequence;
S6022, aligning, by taking any one of the detected base sequences in the second set of sequences as the reference sequence, other detected base sequences in the second set of sequences to the reference sequence, and determining, based on the alignment result, a third set of sequences aligned successfully to the reference sequence; and
S6023, separating the third set of sequences and repeating S6022 one or more times until the second set of sequences comprises 0 detected base sequences, so as to acquire the alignment result.

10. The method according to any one of claims 1-5, wherein S60 comprises:

determining base differences between the detected base sequence at the basic unit position and the detected base sequences at surrounding (k × k - 1) basic unit positions thereof, and determining, based on all the base differences, a score of the basic unit position, wherein k is an odd number greater than 1; and

performing, based on the score of the basic unit position, the clustering;

optionally, the basic unit and the surrounding (k × k - 1) basic units thereof constitute a K × K pixel matrix in the sequencing image with the basic unit located at the center of the K × K pixel matrix;

optionally, performing, based on the score of the basic unit position, the clustering comprises:

if the score of the basic unit position is greater than a third preset value, clustering the detected base sequence at the basic unit position and the detected base sequences at the surrounding (k × k - 1) basic unit positions thereof, or, clustering the basic unit position and the surrounding (k × k - 1) basic unit positions thereof.

11. The method according to any one of claims 1-9, wherein S60 comprises:

determining base differences between the detected base sequence at each basic unit position and the detected base sequences at surrounding (k × k - 1) basic unit positions thereof, and determining, based on the base differences, a score of each basic unit position; and

performing, based on a variation in the score of each basic unit position, the clustering;

optionally, each basic unit and the surrounding (k × k - 1) basic units thereof constitute a K × K pixel matrix in the sequencing image;

optionally, performing, based on a variation in the score of each basic unit position, the clustering comprises:

if a maximum is present in the scores of the basic unit position and the surrounding (k × k - 1) basic unit positions thereof, clustering the basic unit position and the surrounding (k × k - 1) basic unit positions thereof, or, clustering the detected base sequence at the basic unit position and the detected base sequences at the surrounding (k × k - 1) basic unit positions.

12. The method according to any one of claims 1-11, further comprising:

acquiring an image of interest, wherein the image of interest and the image comprising the feature corresponding to the nucleic acid template are from the same field of view;

mapping coordinates of the nucleic acid template with a portion of the base sequence determined based on the clustering result to the image of interest to determine a position of corresponding coordinates in the image of interest;

determining a signal intensity at the position of the corresponding coordinates in the image of interest, the signal intensity being a corrected signal intensity; and

comparing the signal intensity at the position of the corresponding coordinates in the image of interest with a fourth preset value, and determining a base type corresponding to the position based on the comparison result to achieve base calling.

13. An apparatus for determining a base sequence of a nucleic acid template, comprising:

a processing module, configured for processing an image comprising a feature corresponding to the nucleic acid template, comprising: determining a signal intensity at each basic unit in the image, wherein the image comprises a plurality of basic units, the size of the feature corresponding to the nucleic acid template in the image is represented as one or more basic units, and the size of one basic unit is less than or equal to the size of one pixel of the image; and

a detection module, configured for detecting, based on the signal intensity at each basic unit position, the type of one or more bases incorporated into the nucleic acid template corresponding to the basic unit position to determine a detected base sequence at each basic unit position, wherein

the detection module is further configured for clustering, based on a similarity between the detected base sequence at each basic unit position and detected base sequences at surrounding basic unit positions thereof, the detected base sequences or the basic unit positions to determine a portion of the base sequence of the nucleic acid template.

14. A computer device, comprising a memory, a processor, and a computer program stored on the memory and operable on the processor, wherein the processor, when executing the program, implements the method according to any one of claims 1-12.

**15.** A computer-readable storage medium, wherein the medium has a program stored thereon, and the program is executable by a processor to implement the method according to any one of claims 1-12.

Process an image including a feature corresponding to the nucleic acid template, including: determine a signal intensity at each basic unit position in the image — S20

Detect, based on the signal intensity at each basic unit position, the type of one or more bases incorporated into the nucleic acid template corresponding to the basic unit position to determine a detected base sequence at each basic unit position — S40

Cluster, based on a similarity between the detected base sequence at each basic unit position and detected base sequences at surrounding basic unit positions thereof, the detected base sequences or the basic unit positions to determine a portion of the base sequence of the nucleic acid template — S60

**FIG. 1**

Perform, by using a sequencing-by-synthesis method based on surface multi-channel fluorescence microscopic imaging, one or more cycles of sequencing on a plurality of the nucleic acid templates connected to a chip surface to generate one or more corresponding sets of sequencing images, where one set of sequencing images generated by each cycle of sequencing includes a plurality of sequencing images corresponding to four types of bases incorporated into the nucleic acid templates, and the sequencing images have identical resolutions and sizes — S10

Align the one or more sets of sequencing images — S12

Determine, based on a pixel value or a sub-pixel value at the same basic unit position in one or more aligned sets of sequencing images, the signal intensity at the basic unit position of the images — S14

**FIG. 2**

| 1 | 2 | 3 |
| 4 | 5 | 6 |
| 7 | 8 | 9 |

**FIG. 3**

**FIG. 4**

Acquire an image of interest, where the image of interest and the image including the feature corresponding to the nucleic acid template are from the same field of view

501

Mapping coordinates of the nucleic acid template with a portion of the base sequence determined based on the clustering result to the image of interest to determine a position of corresponding coordinates in the image of interest

502

Determine a signal intensity at the position of the corresponding coordinates in the image of interest, the intensity being a corrected intensity

503

Compare the signal intensity at the position of the corresponding coordinates in the image of interest with a fourth preset value, and determine a base type corresponding to the position based on the comparison result to achieve base calling

504

**FIG. 5**

Apparatus for determining a base sequence of a nucleic acid template

Processing module 601

Detection module 602

Alignment module 603

Correction module 604

**FIG. 6**

700

| CPU | 701 | | ROM | 702 | | RAM | 703 |

704

I/O interface 705

| Input section | 706 | | Output section | 707 | | Storage section | 708 | | Communication section | 709 | | Drive | 710 |

| Detachable medium | 711 |

**FIG. 7**

## EUROPEAN SEARCH REPORT

**Application Number**

EP 25 18 5381

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | AU 2020 273 459 A1 (ILLUMINA INC [US]) 7 January 2021 (2021-01-07)<br>* claims 1,29-30,32-35 *<br>* paragraph [0241] - paragraph [0243] *<br>* paragraph [0281] - paragraph [0288] *<br>* paragraph [0335] *<br>* figures 7-11 *<br>----- | 1-15 | INV.<br>G16B30/10<br>G16B40/10<br><br>ADD.<br>C12Q1/6869 |
| X | CN 113 012 757 A (GENEMIND BIOSCIENCES CO LTD) 22 June 2021 (2021-06-22)<br>* paragraph [0003] *<br>* paragraph [0016] *<br>* paragraph [0045] *<br>* paragraph [0047] - paragraph [0052] *<br>* paragraph [0060] - paragraph [0061] *<br>* paragraph [0096] - paragraph [0104] *<br>* paragraph [0127] - paragraph [0129] *<br>* paragraph [0137] - paragraph [0142] *<br>* paragraph [0166] - paragraph [0174] *<br>* paragraph [0181] - paragraph [0187] *<br>----- | 1-15 | |
| X | WO 2023/230278 A2 (ELEMENT BIOSCIENCES INC [US]) 30 November 2023 (2023-11-30)<br>* claim 1 *<br>* figures 5,23 *<br>----- | 1-15 | TECHNICAL FIELDS SEARCHED (IPC)<br><br>G16B<br>C12Q |

The present search report has been drawn up for all claims

| Place of search | Date of completion of the search | Examiner |
|---|---|---|
| Munich | 15 October 2025 | Martínez Cebollada |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
...........................................................................................
& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 25 18 5381

This annex lists the patent family members relating to the patent documents cited in the above-mentioned European search report.
The members are as contained in the European Patent Office EDP file on
The European Patent Office is in no way liable for these particulars which are merely given for the purpose of information.

15-10-2025

| Patent document cited in search report | Publication date | Patent family member(s) | | Publication date |
|---|---|---|---|---|
| AU 2020273459 A1 | 07-01-2021 | AU | 2020273459 A1 | 07-01-2021 |
| | | AU | 2020276115 A1 | 07-01-2021 |
| | | CA | 3104851 A1 | 19-11-2020 |
| | | CA | 3104854 A1 | 19-11-2020 |
| | | CN | 112313750 A | 02-02-2021 |
| | | CN | 112368567 A | 12-02-2021 |
| | | CN | 117935916 A | 26-04-2024 |
| | | EP | 3969884 A1 | 23-03-2022 |
| | | EP | 3970151 A1 | 23-03-2022 |
| | | EP | 4394778 A2 | 03-07-2024 |
| CN 113012757 A | 22-06-2021 | CN | 113012757 A | 22-06-2021 |
| | | EP | 4116402 A1 | 11-01-2023 |
| | | US | 2023027811 A1 | 26-01-2023 |
| | | WO | 2021120715 A1 | 24-06-2021 |
| WO 2023230278 A2 | 30-11-2023 | AU | 2023277548 A1 | 19-12-2024 |
| | | CN | 119585800 A | 07-03-2025 |
| | | EP | 4533460 A2 | 09-04-2025 |
| | | KR | 20250028294 A | 28-02-2025 |
| | | US | 2025285709 A1 | 11-09-2025 |
| | | WO | 2023230278 A2 | 30-11-2023 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 112288783 B